(19)

**Europäisches Patentamt**
**European Patent Office**
**Office européen des brevets**

(11) **EP 4 563 140 B1**

(12) **EUROPEAN PATENT SPECIFICATION**

(45) Date of publication and mention
of the grant of the patent:
**20.05.2026 Bulletin 2026/21**

(21) Application number: **23386125.1**

(22) Date of filing: **28.11.2023**

(51) International Patent Classification (IPC):
*A61K 8/34* (2006.01)  *A61Q 19/00* (2006.01)
*A61K 8/9789* (2017.01)  *A61K 8/9767* (2017.01)
*A61K 8/64* (2006.01)  *A61K 8/44* (2006.01)
*B01D 11/02* (2006.01)

(52) Cooperative Patent Classification (CPC):
**A61K 8/9789; A61K 8/345; A61K 8/44; A61K 8/64;**
**A61K 8/9767; A61Q 19/00; B01D 11/0288**

(54) **EUTECTIC SOLVENT FOR EXTRACTION OF PLANT MATERIAL**

EUTEKTISCHES LÖSUNGSMITTEL ZUR EXTRAKTION VON PFLANZENMATERIAL

SOLVANT EUTECTIQUE POUR L'EXTRACTION DE MATIÈRE VÉGÉTALE

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(43) Date of publication of application:
**04.06.2025 Bulletin 2025/23**

(73) Proprietors:
- **Korres S.A. Natural Products**
  **14452 Metamorfosi (GR)**
- **National Technical University of Athens**
  **10682 Athens (GR)**
- **Hellenic Agricultural Organization-Dimitra**
  **(Elgo-Dimitra)**
  **11145 Athens (GR)**
- **NATIONAL CENTER FOR SCIENTIFIC**
  **RESEARCH**
  **"DEMOKRITOS"**
  **153 41 Agia Paraskevi (GR)**
- **Detsi, Anastasia**
  **15780 Athens (GR)**
- **Tzani, Andromachi**
  **15780 Athens (GR)**
- **Kostopoulou, Ioanna**
  **15780 Athens (GR)**
- **Mamma, Diomi**
  **15780 Athens (GR)**
- **Kalantzi, Styliani**
  **15780 Athens (GR)**
- **Lemoni, Zafeiria**
  **15780 Athens (GR)**
- **Maloupa, Eleni**
  **54644 Thessaloniki (GR)**

- **Grigoriadou, Katerina**
  **55535 Thessaloniki (GR)**
- **Ganopoulos, Ioannis**
  **54352 Pilea, Thessaloniki (GR)**
- **Papanastasi, Katerina**
  **55132 Kalamaria, Thessaloniki (GR)**

(72) Inventors:
- **Stavropoulos, Georgios**
  **13121 Ilion (GR)**
- **Angeli, Konstantina**
  **15561 Cholargos (GR)**
- **Stamatogianni, Chrysoula**
  **14572 Drossia (GR)**
- **Angeli, Maria**
  **16346 Ilioupolis (GR)**
- **Zisimou, Sofia**
  **34132 Halkida (GR)**
- **Touli, Afroditi**
  **14671 Nea Erythraia (GR)**
- **Alexiou, Lida**
  **15232 Halandri (GR)**
- **Michopoulou, Anastasia**
  **19007 Marathonas (GR)**
- **Detsi, Anastasia**
  **15780 Athens (GR)**
- **Tzani, Andromachi**
  **15780 Athens (GR)**
- **Kostopoulou, Ioanna**
  **15780 Athens (GR)**
- **Mamma, Diomi**
  **15780 Athens (GR)**
- **Kalantzi, Styliani**
  **15780 Athens (GR)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **Lemoni, Zafeiria**
  **15780 Athens (GR)**
- **Maloupa, Eleni**
  **54644 Thessaloniki (GR)**
- **Grigoriadou, Katerina**
  **55535 Thessaloniki (GR)**
- **Ganopoulos, Ioannis**
  **54352 Pilea, Thessaloniki (GR)**
- **Papanastasi, Katerina**
  **55132 Kalamaria, Thessaloniki (GR)**
- **Kletsas, Dimitrios**
  **15342 Agia Parakevi (GR)**
- **Pratsinis, Charalampos**
  **11473 Athens (GR)**
- **Mavrogonatou, Eleni**
  **15341 Agia Paraskevi (GR)**
- **Papadopoulou, Adamantia**
  **14563 Kifissia (GR)**

(74) Representative: **Roukounas, Dimitrios**
**c/o Regus Business Center**
**Nymphenburger Straße 4**
**80335 München (DE)**

(56) References cited:
**WO-A2-2011/063080**    **CN-A- 116 602 901**

- **KRISANTI ELSA ANISA ET AL: "Formulation and characterization of betaine-based deep eutectic solvent for extraction phenolic compound from spent coffee grounds", vol. 2175, 1 January 2019 (2019-01-01), NEW YORK, US, pages 020040, XP093150682, ISSN: 0094-243X, Retrieved from the Internet <URL:https://pubs.aip.org/aip/acp/article-pdf/doi/10.1063/1.5134604/14199914/020040_1_online.pdf> DOI: 10.1063/1.5134604**
- **MAKKLIANG FONTHIP ET AL: "Biocompatible natural deep eutectic solvent-based extraction and cellulolytic enzyme-mediated transformation of Pueraria mirifica isoflavones: a sustainable approach for increasing health-bioactive constituents", vol. 8, no. 1, 1 December 2021 (2021-12-01), XP093150805, ISSN: 2197-4365, Retrieved from the Internet <URL:https://bioresourcesbioprocessing.springeropen.com/counter/pdf/10.1186/s40643-021-00428-9.pdf> DOI: 10.1186/s40643-021-00428-9**

Remarks:
The file contains technical information submitted after the application was filed and not included in this specification

**Description**

**FIELD OF THE INVENTION**

The present invention relates to a solvent for the extraction of plant material, in particular for cosmetic purposes.

**BACKGROUND OF THE INVENTION**

Plant extracts are commonly used in the cosmetics industry and are incorporated in numerous formulations to provide specific activities (for example antioxidant, anti-ageing, anti-inflammatory etc).

The solvents used for the extractions are usually organic solvents (alcohols, acetone, hexane), which are commonly of petrochemical origin and require specific handling due to their volatility and flammability. Thus, organic solvents should be avoided in the extraction processes on an industrial scale.

Water or a mixture of water and biobased glycerol or ethanol can often be applied as alternative extraction solvents, especially in the cosmetics industry however, the presence of water requires the addition of a microbiological preservative in order to ensure the stability and preservation of the extracts.

The molecules extracted from plants (phenolic compounds, flavonoids, tannins, terpenes etc) are sensitive to air, oxygen and heat, therefore the obtained extract may have low stability and shelf-life. This fact raises the need for an extraction process that will preserve the sensitive molecules and, ideally, will be able to stabilize them. In addition, the majority of the molecules that are extracted from plants have low to negligible aqueous solubility which impedes their incorporation in water-based cosmetic formulations.

Thus, there is a growing need to find environmentally friendly solvents, ideally biobased with low flammability and high extracting ability, which can be used on an industrial scale and possess the ability to efficiently extract molecules from plants under mild conditions.

Eutectic Solvents (such as Deep Eutectic Solvents, DES) are mixtures comprising two or more components, at least one Hydrogen Bond Donor and one Hydrogen Bond Acceptor, which interact via hydrogen and Van der Waals bonds formation to produce a eutectic mixture which possesses a melting point much lower than the melting point of each component.

In 2011 Choi et al. (Plant Physiol. 2011 Aug; 156(4): 1701-1705) observed that plants under stress (drought or extreme temperatures) produced a high quantity of primary metabolites (amino acids, polyols, organic acids and quaternary ammonium salts). They explained this phenomenon as the formation of a "third liquid" phase in the plant (i.e., deep eutectic systems comprising the primary metabolites) and they named this system Natural Deep Eutectic Solvents (NADES). They postulated that NADES may play a role in all kinds of cellular processes, explaining mechanisms and phenomena that are otherwise difficult to understand, such as the biosynthesis of non-water-soluble small molecules and macromolecules.

NADES have been characterized as the green solvents of the 21st century (Paiva et al. ACS Sustainable Chem. Eng. 2014, 2, 5, 1063-1071). They possess favorable properties and advantages over common organic solvents such as low vapor pressure, high extractive capacity, low production costs, low toxicity and high biodegradability.

The extended hydrogen bond array formed among the molecules of a NADES creates a unique medium, ideal for the extraction of bioactive compounds (Skarpalezos D., Detsi A. Applied Sciences 2019, 9 (19) 4169). Moreover, the NADES environment acts as "storage medium" for the air- and light-sensitive bioactive compounds, protecting them from degradation. One additional characteristic feature of NADES as extraction solvents is that by carefully selecting the components of the NADES one can achieve enhanced bioactivity of the final extract: unlike the case of all the other extraction solvents, which have to be removed from the extract after the process, NADES can be retained and the as-obtained extract (NADES containing the bioactive compounds) has high added value, stemming from the combination of the bioactivity of the NADES's components and the extracted bioactive molecules.

The molecules that are extracted from plants are usually linked to the polysaccharides of the plant cell wall either by hydrogen bonds or by being encapsulated in hydrophobic pockets, resulting in a stable complex. Therefore, the use of enzymes capable of disrupting these complexes and release the bioactive molecules has emerged as a promising, greener alternative to conventional extraction methods (Enzyme Assisted Extraction, EAE).

Plant cell walls consist of a series of complex structural polysaccharides such as cellulose, hemicellulose and pectin, as well as lignin (an aromatic polymer) and even proteins. This structure is what confers cells the stability and resistance to the extraction of the intracellular components. Bioactive components of the plants are present in the cytoplasm or are usually linked to the cell wall polysaccharides either by hydrogen bonds or by being encapsulated in hydrophobic pockets, resulting in a stable complex. Thus, enzymes with specific hydrolytic activities (cellulases, hemicellulases, ligninases, pectinases, proteases) can be used to rupture this matrix in order to gain access to the bioactive components from within the cytosolic spaces and even those bound to the cellular walls.

Hence, the enzyme-assisted extraction manages to release the phenolic compounds, rendering them more easily extractable, improving their extraction rate, selectivity and yield. Moreover, the use of different enzymes, as well as different extraction conditions, strongly affects the outcome, enabling the development of a tailor-made extraction formula to obtain an added-value final product (Pontillo A.R.N.et al. Appl. Sci. 2021, 11, 3724, https://doi.org/10.3390/app11083724).

Ricarte et al. (3Biotech, (2020) 10(9):405) disclose the extraction of carotenoids from sunflower wastes (petals and florets) using hydrophobic NADES (d,l-menthol/ d,l-lactic acid 2:1 as well as other acids such as acetic acid and lauric acid) and the multi-enzyme complex Viscozyme®. However, the use of hydrophobic NADES containing acids as the hydrogen bond donors renders the NADES acidic which is not always a desirable feature for cosmetic applications.

Makkliang et al. (Bioresour. Bioprocess. (2021) 8:76) disclose a combination of a cellulolytic enzyme preparation (from *Trichoderma reesei*) (262 mU/mL) with the NADES choline chloride:propylene glycol (1:2 mol ratio, ChCl:PG) in order to extract daidzin and genistin from *Pueraria mirifica* and simultaneously hydrolyze them to their aglycones daidzein and genistein. However, choline salts, e. g. choline chloride, and choline esters, are prohibited in cosmetic products by Regulation (EC) No 1223/2009 and therefore choline chloride cannot be a component of NADES for cosmetic applications.

Krisanti et al. (AIP Conference Proceedings, (2019), 2175, 020040) discloses an extraction solvent for extracting phenolic compounds from spent coffee grounds comprising a deep eutectic solvent (DES), wherein the DES is a mixture of trimethylglycine and 1,3-propanediol in a molar ratio 1:7. The extraction solvent does not contain water.

CN116602901 discloses an extraction solvent for the preparation of orange peel extract, wherein the solvent consists of trimethylglycine, lactic acid, ethanol, and 1,2-propanediol in a mass ratio of 4:2:5:1. The extraction solvent does not contain water.

WO2011063080 discloses a multicellulase enzyme composition for the enzymatic hydrolysis of cellulose in an aqueous slurry, wherein the composition comprises a cellobiohydrolase, an endoglucanase and a β-glucosidase. Use of this enzyme composition with solvents other than water is not mentioned.

## SUMMARY OF THE INVENTION

The present invention provides a solvent for the extraction of a cosmetically active ingredient from plant material, wherein the extraction solvent comprises a Eutectic Solvent (ES), water and a cellulase, and wherein the eutectic solvent comprises trimethylglycine and 1,3-propanediol.

The extraction solvent of the present invention comprises water, a cellulase and a ES, wherein the ES consists of trimethylglycine and 1,3-propanediol. The extraction solvent of the present invention can provide a liquid extract with high content in cosmetically active compounds. Furthermore, the extraction solvent provides antimicrobial preservation, which means that the liquid extract can be stored as obtained after the extraction and the filtration process without the addition of an additional preservative and without the removal of the extraction solvent. Moreover, the extraction solvent provides stability to the cosmetically active ingredients. In addition, the extraction solvent can be used on an industrial scale for the extraction of plant material and in the preparation of a cosmetic composition.

The present invention provides also a process for the production of an extraction solvent.

In addition, the present invention provides a liquid extract which comprises an extraction solvent and one more cosmetically active ingredients which have been extracted with the extraction solvent from plant material.

The present invention provides also a cosmetic formulation incorporating a liquid extract as obtained from the extraction process without removing the extraction solvent.

## DETAILED DESCRIPTION OF THE INVENTION

The present invention provides an extraction solvent which can (a) efficiently extract cosmetically active ingredients from plant material; (b) act as a stabilizing medium for the protection of the cosmetically active ingredients from photodegradation and oxidation; (c) be self-preserved, meaning that no addition of extra preservatives is required for the antimicrobial preservation of the liquid extract; (d) be incorporated in a cosmetic formulation; (e) be used on an industrial scale.

Thus, the present invention provides an extraction solvent for the extraction of a cosmetically active ingredient from plant material, wherein the solvent comprises

a) a eutectic solvent in a concentration from 72% w/w to 82% w/w of the extraction solvent, wherein the eutectic solvent consists of trimethylglycine and 1,3-propanediol in a molar ratio of 1:7,

b) water in a concentration from 17% w/w to 27% w/w of the extraction solvent, and

c) a cellulase in a concentration from 0,005 % w/w to 0,050 % w/w of the extraction solvent.

Preferably, the concentration of the eutectic solvent in the extraction solvent is from 75% w/w to 79% w/w. More preferably, the concentration of the eutectic solvent in the extraction solvent is from 76% w/w to 78% w/w.

Preferably, the concentration of water in the extraction solvent is from 21% w/w to 25% w/w. More preferably, the concentration of water in the extraction solvent is from 22% w/w to 24% w/w.

Preferably, the concentration of cellulase in the extraction solvent is from 0,007% w/w to 0,040% w/w. More preferably, the concentration of cellulase in the extraction solvent is from 0,008% w/w to 0,035% w/w.

Throughout the present application, the term % w/w, means grams of the eutectic solvent, or of a component (e.g., water, cellulase) per 100 grams of the extraction solvent, unless specified otherwise.

Trimethylglycine is a chemical compound also known as glycine betaine, or betaine. The terms "trimethylglycine" and "betaine" are used interchangeably throughout the present application.

The term "cellulase" refers to an enzyme which catalyzes cellulolysis, i.e., the decomposition of cellulose into monosaccharides, oligosaccharides, and/or shorter polysaccharides. The cellulase may be one enzyme of a mixture of enzymes which catalyze cellulolysis.

According to a preferred embodiment, the cellulase is a mixture of endoglucanase, cellobiohydrolase κα β-glucosidase. An example of a commercially available product which comprises a mixture of endoglucanase, cellobiohydrolase κα β -glucosidase is the product Celluclast ®, which is an aqueous solution of the three enzymes having density 1,1-1,3g/mL and enzymatic activity >=700 U/g.

The extraction solvent of the present invention may be used for the extraction of a cosmetically active ingredient from a plant. Examples of plants which can be used in the extraction include species of the Families: Asteraceae, Rosaceae, Lamiaceae, Vitaceae, Iridaceae, Oleaceae, Pinaceae, Rutaceae, Rutaceae, Cactaceae, Fabaceae

Preferably, the plant is selected from species of the Genus: *Helichrysum, Pinus, Rosa, Sideritis Helianthus, Calendula, Prunus Vitis Crocus , Olea, Pinus, Opuntia, Achillea, Ceratonia, Lavandula*

More preferably, the plant is selected from the species: *Salvia officinalis, Salvia fruticose, Helichrysum italicum, Helichrysum amorginum, Helichrysum orientale , Thymus voulgaris, Calendula officinalis, Coridothymus capitatus, Vitis vinifera, Crocus sativus. Olea europaea, Citrus medica, Lavandula officinalis, Ceratonia silique, Prunus dulcis. Achillea millefolium, Helianthus annuus, Rosa canina, Rosa damascene, Sideritis scardica, Sideritis syriaca, Sideritis euboea, Pinus heldreichii, Pinus nigra, Opuntia ficus-indica.*

The term "plant material" refers to any part of a plant, such as roots, bark, flowers, leaves, stems, which contains a cosmetically active ingredient.

A "cosmetically active ingredient" is a compound which can be used as active ingredient in a cosmetic composition. Examples of cosmetically active ingredients include phenolic acids, phenolic acid esters, flavonoids, secoiridoids, stilbenes, phenolic alcohols, carotenoids, alkaloids, lipids, phenylpropanoids, fragrances, pigments, and terpenoids including saponins.

All components of the extraction solvent of the present invention are compatible with the technical and regulatory requirements specific to the cosmetic field and cosmetic compositions comprising them.

A person skilled in the art understands that 100g of the ES trimethylglycine and 1,3-propanediol in a molar ratio 1:7 corresponds to mass proportions of trimethylglycine / 1,3-propanediol 39,4g:60,6g.

The present invention provides also a process for the production of an extraction solvent as defined above, wherein the process comprises

i) preparing a eutectic solvent consisting of trimethylglycine and 1,3-propanediol in a molar ratio of 1:7 by mixing trimethylglycine and 1,3-propanediol at 50 °C to 70 °C until a clear solution is formed,
ii) adding water to the eutectic solvent,
iii) adding a cellulase to the solution obtained in b) to obtain the extraction solvent.

Preferably, step i) is carried out at 55 °C to 65 °C. More preferably, step i) is carried out at 58 °C to 62 °C.

Preferably, steps ii) and iii) are carried out at 20 °C to 25 °C.

The present invention provides also the use of the extraction solvent of the present invention in the extraction of a cosmetically active ingredient from plant material.

The extraction can be carried out using various processes which are well known in the art.

An example of an extraction process using the extraction solvent of the present invention is as follows: the plant material is subjected to grinding using a cutting machine prior to use. Then the plant material is added to a round bottomed flask and the extraction solvent is added. Preferably, the plant material/extraction solvent weight ratio ranges from 0,5:100 to 10:100. More preferably, the plant material/extraction solvent weight ratio ranges from 3:100 to 8:100. Most preferably, the plant material/extraction solvent weight ratio is 6:100. The extraction is performed by magnetically stirring the mixture and heating at a temperature from 30°C to 60°C. More preferably the heating temperature is from 40°C to 50 °C. Most preferably the heating temperature is 45°C. The extraction time can vary from 2h to 24h. More preferably, the extraction time is between 2h and 6h. Most preferably, the extraction time is 4h.

The extraction solvent of the present invention exhibits excellent extraction performance, which means that it can extract large amounts of cosmetically active ingredients from a wide variety of plant material. Furthermore, although the concentration of water in the extraction solvent is low, it has surprisingly been found that cellulase exhibits its cellulolytic activity in the solvent. In addition, the extraction solvent can be used for extractions from plant material on an industrial scale.

The present invention provides also a liquid extract as obtained from the extraction process, without the removal of the extraction solvent. Thus, the present invention provides a liquid extract comprising the extraction solvent of the present invention and a cosmetically active ingredient which has been extracted with the extraction solvent from plant material. The liquid extract exhibits excellent stability of the cosmetically active ingredient. Furthermore, the liquid extract, i.e., the extraction solvent, exhibits excellent antimicrobial preservation, which means that it can be stored for long periods of time without the addition of an additional preservative.

An additional advantage of the extraction solvent and the liquid extract of the present invention is that it has favorable physical properties that enable its use on an industrial scale. For example, one of the problems frequently encountered when extractions are carried out on industrial scale is the inability of the extraction medium to pass through sterilizing depth filter sheets of 0,2-0,4$\mu$m pore diameters. The main components of these filters are cellulose, natural diatomaceous earth and perlite. The extraction solvent (and therefore the liquid extract) of the present invention can easily pass through sterilizing filters. In addition, the extraction solvent (and therefore the liquid extract) exhibits excellent antimicrobial preservation properties as confirmed by its low water activity ($a_w$), which means that it can be stored for long periods of time without the need of addition of an additional preservative

The present invention provides also a cosmetic composition comprising of the liquid extract of the present invention and a cosmetically acceptable carrier. The cosmetic composition may have different forms & types, such as creams, emulsions, oils, lotions, gels, skin-whitening products, anti-wrinkle products, face masks, hand soaps, deodorants, anti-perspirants, hair care products, hair conditioning products, shaving products (creams, foams, lotions), sunscreen products, perfumes, oily based products (such oily cleansing balms, body oils), rinse-off products such as shower gels or face cleansers, products for external intimate hygiene, sunscreens, make-up and products removing make-up, self-tanning products.

A typical cosmetic product comprises a number of cosmetically acceptable excipients, such as solvents, emulsifiers, chelating agents, viscosity enhancers, film formers, antioxidants, thickening agents, synthetic & natural oils/waxes, emollients, preservatives, fragrance, pH adjusters, surfactants, foam boosters, sun filters, spf boosters, depending on the nature of the final cosmetic product.

A very well-known & typical cosmetic format is an emulsion, which is subsequently categorized as either an oil-in-water (o/w) or a water-in-oil (w/o) emulsion.

In w/o emulsions, oil is dispersed in a continuous water phase, while in w/o emulsions, water droplets are dispersed in oil.

The most frequently marketed emulsions are the O/W type, which normally comprise two phases. The aqueous phase typically includes the water, viscosity enhancers, film formers, stabilizers etc, whereas the oily phase typically comprises a mixture of waxes, oils, emollients, skin conditioning agents, antioxidants, emulsifiers etc. An emulsifier, one of the most crucial components of an emulsion, is a surfactant with a lipophilic and a hydrophilic part, and is required to 'bind' the two phases and create the emulsion. It mixes and disperses the oily in aqueous phase and vice versa. Emulsifiers are characterized by an HLB (Hydrophilic-Lipophilic Balance) value, which expresses the relationship between the hydrophilic and lipophilic part of their molecule and ranges from 0 to 20. An O/W emulsion also typically includes active ingredients, that may have whitening, antiaging, moisturizing properties for example (depending on the desired marketing claims of the end product), preservatives, fragrance, pH adjusters.

In some cases, emulsion in W/O form can be introduced. In this particular emulsion type there is an external phase; a continuous oil phase, and also an aqueous phase, that is dispersed in the form of small droplets, into the external oily phase.

The properties of the final cosmetic product are typically different compared to the O/W form in terms of the cream thickness/texture/absorption/after-feel effect.

Examples of solvents include water, alcohol, or other type of liquids depending on the nature of the final cosmetic product.

Examples of emulsifiers include sorbitan stearate, glyceryl stearate citrate.

Examples of preservative include sodium benzoate, potassium sorbate.

Examples of pH adjusters include citric acid, lactic acid.

Examples of thickeners include xathan gum, polysorbates.

Examples of chelating agents include sodium phytate, sodium gluceptate

EXAMPLES

Example 1: Extraction of Black Pine (*Pinus nigra*) conifers

**Preparation of the eutectic solvent Betaine/ 1,3-propanediol 1:7 (molar ratio)**

The eutectic solvent is prepared by mixing betaine (0,15mol, 17,5g) and 1,3-propanediol (1,05mol, 79,8g) in a round-bottomed flask. The mixture is stirred at 60°C for 1h until a clear solution is formed.

**Preparation of the extraction solvent**

To 77g of the eutectic solvent Betaine/ 1,3-propanediol 1:7 (molar ratio) is added 23g of water and to this solution is added Celluclast® in a concentration of 0.1%w/w relative to the extraction solvent.

Celluclast® is a mixture of endoglucanase, cellobiohydrolase κα β-glucosidase produced by *Trichoderma reesei* (deifined as "cellulase produced by *Trichoderma reesei*" in the Product Specification Sheet) and the main activity of the preparation is cellulolytic. The enzyme preparation is an aqueous solution with density 1.1-1.3g/mL and enzymatic activity >=700 U/g comprising 10 - 20 % w/w cellulase.

The final solution is referred to hereinbelow as the extraction solvent.

**Extraction procedure**

Black Pine (*Pinus nigra*) conifers were subjected to grinding using a domestic multi-cutting machine prior to use.

The plant material was placed in a round bottomed flask and the extraction solvent was added so as to achieve a plant material/ extraction solvent weight ratio 6:100. The mixture was magnetically stirred at 45°C for 4h.

The mixture was then centrifuged at 9000rpm for 15min and the supernatant was collected and stored in the refrigerator.

The supernatant is referred to hereinbelow as the liquid extract.

**Colorimetric Determination of Total Phenolic Content of Liquid Extracts (TPC)**

A stock aqueous solution 15% v/v of the liquid extract was prepared. 40 μL of the stock solution were mixed with 2 mL of ultrapure water and 200 μL of Folin-Ciocalteau reagent, and the mixture was stirred using a Vortex mixer before being incubated in the dark for 5 min. Then, 600 μL of saturated aqueous $Na_2CO_3$ solution were added for pH control, the mixture was stirred again and the final volume of the solution was adjusted to 4 mL by adding ultrapure water. After 1 h incubation in the dark at room temperature, the absorbance was measured at 755 nm using a microplate reader. The above-mentioned procedure was repeated for a blank sample, which contained water, and its absorbance was subtracted from that of each sample. The experiments were performed in triplicate.

The TPC of the extracts was calculated as gallic acid equivalents (GAE) from a calibration curve, using gallic acid as a standard and it was expressed as mg GAE per g of plant material.

$$TPC \left( \frac{mg\ GAE}{g\ plant\ material} \right) = \frac{C_{GA} \left( \frac{mg}{mL} \right) * D * V_{ext}\ (mL)}{m_{plant\ material}\ (g)},$$

where $C_{GA}$ is the calculated GAE by calibration curve, D is the dilution factor of the extract, $V_{ext}$ is the extraction solvent volume used for the extraction and $m_{plant\ material}$ is the mass of used plant material for the extraction.

**Colorimetric Determination of Total Flavonoid Content of Liquid Extracts (TFC)**

A stock aqueous solution 15% v/v of the liquid extract was prepared. 100 μL of the stock solution were mixed with 60 μL 5 % aqueous $NaNO_2$ solution. The mixture was allowed to remain for 6 min at room temperature. Then 120 μL 10 % aqueous $AlCl_3$ solution were added and the mixture was incubated for 5 min. Then 600 μL aqueous NaOH solution (1M) were added and the final volume of the solution was adjusted to 2 mL by adding ultrapure water. After 15 min incubation in the dark at room temperature, the absorbance was measured at 500 nm using microplate reader.

The TFC of the extracts was calculated as catechin equivalents (CATE) from a calibration curve, using catechin as a standard and it was expressed as mg CATE per g of plant material.

$$TFC \left( \frac{mg\ CATE}{g\ plant\ material} \right) = \frac{C_{CATE} \left( \frac{mg}{mL} \right) * D * V_{ext}\ (mL)}{m_{plant\ material}\ (g)},$$

where $C_{CATE}$ is the calculated CATE by calibration curve, D is the dilution factor of the extract, $V_{ext}$ is the extraction solvent volume used for the extraction and $m_{plant\ material}$ is the mass of used plant material for the extraction.

**Results**

In addition to the extraction described above, extractions without the addition of the enzyme, using the eutectic solvent Betaine / 1,3-propanediol 1:7 (molar ratio) in which water was added so that the weight ratio of the eutectic solvent to water is 77:23, were performed under the same experimental conditions (plant material/solvent weight ratio 6:100, magnetic stirring at 45°C for 4h). For the laboratory scale experiments, the amount of extraction solvent was 15g and the amount of plant material was 0,9g.

The mixture was then centrifuged at 9000rpm for 15min and the supernatant was collected and stored in the refrigerator.

In addition to the extraction described above, extractions using only 1,3-propanediol were performed under the same experimental conditions (plant material/solvent weight ratio 6:100, magnetic stirring at 45°C for 4h). The mixture was then centrifuged at 9000rpm for 15min and the supernatant was collected and stored in the refrigerator.

The Total Phenolic Content (TPC) and Total Flavonoid Content (TFC) of the Black pine (*Pinus nigra*) conifers extracts were determined as described above. The results are shown in Table 1.

**Table 1** - **Total Phenolic Content (TPC) and Total Flavonoid Content (TFC) of the Black pine (*Pinus nigra*) conifers extracts (Extraction conditions: extraction time 4h; extraction temperature 45°C)**

| Solvent | TPC (mg gallic acid/g plant material) | TFC (mg catechin/g plant material) |
|---|---|---|
| Betaine/ Zemea® 1:7 (molar ratio) - water - Celluclast® ("extraction solvent") | 59,42 ± 0,35 | 50,96 ± 1,70 |
| Betaine / Zemea® 1:7 (molar ratio) - w/w water | 36,23 ± 0,90 | 29,73 ± 0,90 |
| Zemea® | 20,61 ± 0,41 | 16,11 ± 0,07 |
| Zemea®: 1,3-propanediol | | |

The above results show that when the eutectic solvent Betaine (trimethylglycine) / 1,3-propanediol 1:7 (molar ratio) is used in combination with a cellulolytic enzyme and water, a significant improvement in the extraction performance is observed.

Example 2: Extraction of *Rosa canina* fruit shells

The extraction solvent was prepared as described in Example 1 above.

Extraction procedure

*Rosa canina* fruit shells were subjected to grinding using a domestic multi-cutting machine prior to use.

The plant material was placed in a round bottomed flask and the extraction solvent was added so as to achieve a plant material/extraction solvent weight ratio 6:100. The mixture was magnetically stirred at 45°C for 4h. For the laboratory scale experiments, the amount of extraction solvent was 15g and the amount of plant material was 0,9g.

The mixture was then centrifuged at 9000rpm for 15min and the supernatant was collected and stored in the refrigerator.

Extractions without the enzyme, using the eutectic Betaine / 1,3-propanediol 1:7 (molar ratio) in which water was added so that the weight ratio of the eutectic solvent to water 77:23,, were performed under the same experimental conditions (plant material/solvent weight ratio 6:100, magnetic stirring at 45°C for 4h). The mixture was then centrifuged at 9000rpm for 15min and the supernatant was collected and stored in the refrigerator.

In addition to the extraction described above, extractions using only 1,3-propanediol were performed under the same experimental conditions (plant material/solvent weight ratio 6:100, magnetic stirring at 45°C for 4h). The mixture was then centrifuged at 9000rpm for 15min and the supernatant was collected and stored in the refrigerator.

The Total Phenolic Content (TPC) and Total Flavonoid Content (TFC) of the *Rosa canina* fruit shells extracts were determined as described in Example 1 above. The results are shown in Table 2.

**Table 2 - Total Phenolic Content (TPC) and Total Flavonoid Content (TFC) of the *Rosa canina* fruit shells extracts (Extraction conditions: extraction time:4h; extraction temperature 45°C)**

| Solvent | TPC (mg gallic acid/g plant material) | TFC (mg catechin/g plant material) |
|---|---|---|
| Betaine/ Zemea® 1:7 (molar ratio) - water - Celluclast® ("extraction solvent") | 80,88 ± 1,09 | 54,47 ± 2,76 |
| Betaine / Zemea® 1:7 (molar ratio) - water | 66,75 ± 1,21 | 47,79 ± 0,71 |
| Zemea® | 30,24 ± 0,72 | 20,18 ± 0,60 |
| Zemea®: 1,3-propanediol | | |

The above results show that when the eutectic solvent Betaine (trimethylglycine) / 1,3-propanediol 1:7 (molar ratio) is used in combination with a cellulolytic enzyme and water, a significant improvement in the extraction performance is observed.

**Example 3: Extraction of *Helichrysum amorginum* flowers**

The extraction solvent was prepared as described in Example 1 above.

**Extraction procedure**

*Helichrysum amorginum* flowers were subjected to grinding using a domestic multi-cutting machine prior to use.
The plant material was placed in a round bottomed flask and the extraction solvent was added so as to achieve a plant material/extraction solvent weight ratio 6:100. The mixture was magnetically stirred at 45°C for 4h. For the laboratory scale experiments, the amount of extraction solvent was 15g and the amount of plant material was 0.9g.
The mixture was then centrifuged at 9000rpm for 15min and the supernatant was collected and stored in the refrigerator.
Extractions without the enzyme, using the eutectic Betaine / 1,3-propanediol 1:7 (molar ratio) in which water was added so that the weight ratio of the eutectic solvent to water 77:23, were performed under the same experimental conditions (plant material/solvent weight ratio 6:100, magnetic stirring at 45°C for 4h). The mixture was then centrifuged at 9000rpm for 15min and the supernatant was collected and stored in the refrigerator.
In addition to the extraction described above, extractions using only 1,3-propanediol were performed under the same experimental conditions (plant material/solvent weight ratio 6:100, magnetic stirring at 45°C for 4h). The mixture was then centrifuged at 9000rpm for 15min and the supernatant was collected and stored in the refrigerator.
The Total Phenolic Content (TPC) and Total Flavonoid Content (TFC) of the *Rosa canina* fruit shells extracts were determined as described in Example 1 above. The results are shown in Table 3.

**Table 3 - Total Phenolic Content (TPC) and Total Flavonoid Content (TFC) of the *Helichrysum amorginum* flowers extracts (Extraction conditions: extraction time:4h; extraction temperature 45°C)**

| Solvent | TPC (mg gallic acid/g plant material) | TFC (mg catechin/g plant material) |
|---|---|---|
| Betaine/ Zemea® 1:7 (molar ratio) - water - Celluclast ® ("extraction solvent") | 19,06 ± 0,19 | 7,83 ± 0,12 |
| Betaine / Zemea® 1:7 (molar ratio) - water | 14,91 ± 0,34 | 4,21 ± 0,21 |
| Zemea® | 14,72 ± 0,29 | 2,12 ± 0,07 |
| Zemea®: 1,3-propanediol | | |

The above results show that when the eutectic solvent Betaine (trimethylglycine) / 1,3-propanediol 1:7 (molar ratio) is used in combination with a cellulolytic enzyme and water, a significant improvement in the extraction performance is observed.

**Example 4: Extraction on industrial scale**

Liquid extracts of *Pinus nigra* conifers, *Rosa canina* fruit shells & *Helichrysum amorginum* flowers were produced on industrial scale. The produced liquid extracts were proved compatible with the following requirements:

- The produced liquid extracts, i.e., the solution of the extracted compounds in the extraction solvent, can penetrate sterilizing filters with depth filter sheets of 0,2-0,4 $\mu$m pore diameters. The main components of the filters are cellulose, natural diatomaceous earth and perlite.
- All the produced extracts were compliant with the strictest requirements of the European Pharmacopoeia regarding their microbial status so as to be characterized as **self-preserved extracts** with no need for the addition of further preservatives

More specifically, *Pinus nigra* conifers, *Rosa canina* fruit shells & *Helichrysum amorginum* flowers were extracted in a 50 L vessel using the extraction solvent and conditions described in Examples 1, 2 & 3 above.

After filtration of the extracts with depth filter sheets of 0,2-0,4 $\mu$m pore diameters their Water Activity and Density were measured and the results are shown in Table 4 below.

**Table 4**

| Extract | Water Activity ($a_w$) | Density (g/mL) |
|---|---|---|
| *Pinus nigra* conifers | 0,385 | 1,0674 g/ml |
| *Rosa canina* fruit shells | 0,382 | 1,0678 |
| *Helichrysum amorginum* flowers | 0,383 | 1,0675 |

Water activity is a fundamental property of aqueous solutions, and by definition is the ratio of the vapor pressure of the water in the substrate (p) to that of pure water at the same temperature ($p_o$):

$$a_w = p/p_o$$

Water activity is a measure of how efficiently the water present can take part in a chemical (physical) reaction, perform a biochemical transfer or exchange through a semipermeable membrane.

The definition of moisture conditions in which pathogenic or spoilage microorganisms cannot grow is of paramount importance to cosmetic products preservation. Pathogenic microorganisms cannot grow at $a_w < 0.60$.

**Micro-Challenge test**

The microbial stability of filtrated liquid extracts from the industrial scale process was verified by micro-challenge tests. The test protocol is described in European Pharmacopoeia, section 5.1.3 "Efficacy of Antimicrobial Preservation", Edition 11. The test involves challenging a preparation with a prescribed inoculum of suitable microorganisms, storing the inoculated preparation at a prescribed temperature, withdrawing samples from the container at specified intervals of time and counting the microorganisms in the samples so removed.

The results are shown in Tables 5, 6 & 7 below.

**Table 5** *Pinus nigra* conifers extract results from micro-challenge test

| SAMPLE DATA | | | | | |
|---|---|---|---|---|---|
| **PET** | **PRODUCT** | **PRODUCTION/ R&D CODE** | **FORMULATOR** | **START DATE** | **DATE RECEIVED** |
| 10001 | *Pinus nigra* conifers extract | PN.001 | **STAVROPOULOS** | 22/5/2023 | 21/6/2023 |
| **RAW DATA** | | | | | |
| **ATCC STRAIN** | **Inoculum cfu/ml** | **day 2 cfu/ml** | **day 7 cfu/ml** | **day 14 cfu/ml** | **day 28 cfu/ml** |
| *Pseudomonas aeruginosa ATCC 9027 (USP)* | 415000000 | 10 | 10 | 10 | 10 |
| *Staphylococcus aureus ATCC 6538 (USP)* | 185000000 | 10 | 10 | 10 | 10 |
| *Escherichia coli ATCC 8739 (USP)* | 305000000 | 10 | 10 | 10 | 10 |
| *Candida albicans ATCC 10231 (USP)* | 57000000 | 10 | 10 | 10 | 10 |
| *Aspergillus brasiliensis ATCC 16404 [A.niger] (USP)* | 63000000 | 10 | 10 | 10 | 10 |
| *Burkholderia cepacia ATCC 25416* | 100000000 | 10 | 10 | 10 | 10 |
| *Enterobacter cloacae ATCC 13047* | 135000000 | 10 | 10 | 10 | 10 |
| *Pluralibacter (Enterobacter) gergoviae ATCC 33028* | 220000000 | 10 | 10 | 10 | 10 |

**Table 6** *Rosa canina* fruit shells extract results from micro-challenge test

| SAMPLE DATA | | | | | |
|---|---|---|---|---|---|
| **PET** | **PRODUCT** | **PRODUCTION/ R&D CODE** | **FORMULATOR** | **START DATE** | **DATE RECEIVED** |
| 10003 | *Rosa canina fruit shells extract* | RC.001 | **STAVROPOULOS** | 22/5/2023 | 21/6/2023 |
| **RAW DATA** | | | | | |
| **ATCC STRAIN** | **Inoculum cfu/ml** | **day2 cfu/ml** | **day7 cfu/ml** | **day14 cfu/ml** | **day28 cfu/ml** |
| *Pseudomonas aeruginosa ATCC 9027 (USP)* | 415000000 | 10 | 10 | 10 | 10 |
| *Staphylococcus aureus ATCC 6538 (USP)* | 185000000 | 10 | 10 | 10 | 10 |
| *Escherichia coli ATCC 8739 (USP)* | 305000000 | 10 | 10 | 10 | 10 |
| *Candida albicans ATCC 10231 (USP)* | 57000000 | 10 | 10 | 10 | 10 |
| *Aspergillus brasiliensis ATCC 16404 [A.niger] (USP)* | 63000000 | 10 | 10 | 10 | 10 |
| *Burkholderia cepacia ATCC 25416* | 100000000 | 10 | 10 | 10 | 10 |
| *Enterobacter cloacae ATCC 13047* | 135000000 | 10 | 10 | 10 | 10 |

(continued)

| SAMPLE DATA | | | | | |
|---|---|---|---|---|---|
| PET | PRODUCT | PRODUCTION/ R&D CODE | FORMULATOR | START DATE | DATE RECEIVED |
| 10003 | *Rosa canina fruit shells extract* | RC.001 | **STAVROPOULOS** | 22/5/2023 | 21/6/2023 |
| RAW DATA | | | | | |
| **ATCC STRAIN** | Inoculum cfu/ml | day2 cfu/ml | day7 cfu/ml | day14 cfu/ml | day28 cfu/ml |
| ***Pluralibacter (Enterobacter) gergoviae ATCC 33028*** | 220000000 | 10 | 10 | 10 | 10 |

**Table 7** *Helichrysum amorginum* flowers extract micro-challenge test

| SAMPLE DATA | | | | | |
|---|---|---|---|---|---|
| PET | PRODUCT | PRODUCTION/ R&D CODE | FORMULATOR | START DATE | DATE RECEIVED |
| 10004 | *Helichrysum amorginum* flowers extract | HA.001 | **STAVROPOULOS** | 22/5/2023 | 21/6/2023 |
| RAW DATA | | | | | |
| **ATCC STRAIN** | Inoculum cfu/ml | day 2 cfu/ml | day 7 cfu/ml | day 14 cfu/ml | day 28 cfu/ml |
| ***Pseudomonas aeruginosa ATCC 9027 (USP)*** | 415000000 | 10 | 10 | 10 | 10 |
| ***Staphylococcus aureus ATCC 6538 (USP)*** | 185000000 | 10 | 10 | 10 | 10 |
| ***Escherichia coli ATCC 8739 (USP)*** | 305000000 | 10 | 10 | 10 | 10 |
| ***Candida albicans ATCC 10231 (USP)*** | 57000000 | 10 | 10 | 10 | 10 |
| ***Aspergillus brasiliensis ATCC 16404 [A.niger] (USP)*** | 63000000 | 10 | 10 | 10 | 10 |
| ***Burkholderia cepacia ATCC 25416*** | 100000000 | 10 | 10 | 10 | 10 |
| ***Enterobacter cloacae ATCC 13047*** | 135000000 | 10 | 10 | 10 | 10 |
| ***Pluralibacter (Enterobacter) gergoviae ATCC 33028*** | 220000000 | 10 | 10 | 10 | 10 |

The three (3) liquid extracts, produced on industrial scale, successfully passed the challenge tests with the stricter criteria A of European Pharmacopoeia after their filtration with no other addition of preservatives. This is a result of the specific composition of the extraction solvent that simultaneously achieves:

- Values of Density lower than 1,1 g/mL , a condition that permits the equipment of the micro-filtration at industrial scale to work properly.
- Values of $a_w$ much lower than 0,60, which represent an unfavorable environment for microbial development.

**Claims**

1. An extraction solvent for the extraction of a cosmetically active ingredient from plant material, wherein the solvent comprises

a) a eutectic solvent, in a concentration from 72% w/w to 82% w/w of the extraction solvent, wherein the eutectic solvent consists of trimethylglycine and 1,3-propanediol in a molar ratio of 1:7,
b) water, in a concentration from 17% w/w to 27% w/w of the extraction solvent, and
c) a cellulase, in a concentration from 0,005% w/w to 0,050% w/w of the extraction solvent.

2. The extraction solvent according to claim 1, wherein the concentration of the eutectic solvent in the extraction solvent is from 75% w/w to 79% w/w.

3. The extraction solvent according to claim 1 or 2, wherein the concentration of the eutectic solvent in the extraction solvent is from 76% w/w to 78% w/w.

4. The extraction solvent according to any one of the preceding claims, wherein the concentration of water in the extraction solvent is from 21% w/w to 25% w/w.

5. The extraction solvent according to any one of the preceding claims, wherein the concentration of water in the extraction solvent is from 22% w/w to 24% w/w.

6. The extraction solvent according to any one of the preceding claims, wherein the concentration of cellulase in the extraction solvent is from 0,007% w/w to 0,040% w/w.

7. The extraction solvent according to any one of the preceding claims, wherein the concentration of cellulase in the extraction solvent is from 0,008% w/w to 0,035% w/w.

8. The extraction solvent according to any one of the preceding claims, wherein the cellulase is a mixture of endoglucanase, cellobiohydrolase $\kappa\alpha$ $\beta$-glucosidase.

9. Use of the extraction solvent according to in any one of claims 1 to 8 for the extraction of a cosmetically active ingredient from plant material.

10. Use according to claim 9, wherein the plant is selected from *Salvia officinalis, Salvia fruticose, Helichrysum italicum, Helichrysum amorginum, Helichrysum orientale , Thymus voulgaris, Calendula officinalis, Coridothymus capitatus, Vitis vinifera, Crocus sativus. Olea europaea, Citrus medica, Lavandula officinalis, Ceratonia silique, Prunus dulcis. Achillea millefolium, Helianthus annuus, Rosa canina, Rosa damascene, Sideritis scardica, Sideritis syriaca, Sideritis euboea, Pinus heldreichii, Pinus nigra,* or *Opuntia ficus-indica.*

11. A liquid extract comprising the extraction solvent according to any one of claims 1 to 8 and a cosmetically active ingredient which has been extracted with the extraction solvent from plant material.

12. The liquid extract according to claim 11, wherein the cosmetically active ingredient is selected from a phenolic acid, a phenolic acid ester, a flavonoid, a secoiridoid, a stilbene, a phenolic alcohol, a carotenoid, an alkaloid, a lipid, a phenylpropanoid, a fragrance, a pigment, or a terpenoid.

13. A cosmetic composition comprising the liquid extract according to claim 11 or 12 and a cosmetically acceptably carrier.

14. The cosmetic composition according to claim 13, wherein the composition is an emulsion.

15. A process for the preparation of the extraction solvent as defined in any one of claims 1 to 8, wherein the process comprises

i) preparing a eutectic solvent consisting of trimethylglycine and 1,3-propanediol in a molar ratio of 1:7 by mixing trimethylglycine and 1,3-propanediol at 50 °C to 70 °C until a clear solution is formed,
ii) adding water to the eutectic solvent,
iii) adding a cellulase to the solution obtained in b) to obtain the extraction solvent.

**Patentansprüche**

1. Extraktionslösungsmittel zur Extraktion eines kosmetisch wirksamen Inhaltsstoffs aus Pflanzenmaterial, wobei das Lösungsmittel

   a) ein eutektisches Lösungsmittel in einer Konzentration von 72 Gew.-% bis 82 Gew.-% des Extraktionslösungsmittels, wobei das eutektische Lösungsmittel aus Trimethylglycin und 1,3-Propandiol in einem Molverhältnis von 1:7 besteht,
   b) Wasser in einer Konzentration von 17 Gew.-% bis 27 Gew.-% des Extraktionslösungsmittels und
   c) eine Cellulase in einer Konzentration von 0,005 Gew.-% bis 0,050 Gew.-% des Extraktionslösungsmittels umfasst.

2. Extraktionslösungsmittel nach Anspruch 1, wobei die Konzentration des eutektischen Lösungsmittels im Extraktionslösungsmittel 75 Gew.-% bis 79 Gew.-% beträgt.

3. Extraktionslösungsmittel nach Anspruch 1 oder 2, wobei die Konzentration des eutektischen Lösungsmittels im Extraktionslösungsmittel 76 Gew.-% bis 78 Gew.-% beträgt.

4. Extraktionslösungsmittel nach einem der vorhergehenden Ansprüche, wobei die Konzentration von Wasser im Extraktionslösungsmittel 21 Gew.-% bis 25 Gew.-% beträgt.

5. Extraktionslösungsmittel nach einem der vorhergehenden Ansprüche, wobei die Konzentration von Wasser im Extraktionslösungsmittel 22 Gew.-% bis 24 Gew.-% beträgt.

6. Extraktionslösungsmittel nach einem der vorhergehenden Ansprüche, wobei die Konzentration von Cellulase im Extraktionslösungsmittel zwischen 0,007 Gew.-% und 0,040 Gew.-% beträgt.

7. Extraktionslösungsmittel nach einem der vorhergehenden Ansprüche, wobei die Konzentration an Cellulase im Extraktionslösungsmittel zwischen 0,008 Gew.-% und 0,035 Gew.-% beträgt.

8. Extraktionslösungsmittel nach einem der vorhergehenden Ansprüche, wobei die Cellulase eine Mischung aus Endoglucanase, Cellobiohydrolase κα β-Glucosidase ist.

9. Verwendung des Extraktionslösungsmittels nach einem der Ansprüche 1 bis 8 zur Extraktion eines kosmetisch wirksamen Inhaltsstoffs aus pflanzlichem Material.

10. Verwendung nach Anspruch 9, wobei die Pflanze ausgewählt ist aus *Salvia officinalis, Salvia fruticose, Helichrysum italicum, Helichrysum amorginum, Helichrysum orientale, Thymus vulgaris, Calendula officinalis, Coridothymus capitatus, Vitis vinifera, Crocus sativus, Olea europaea, Citrus medica, Lavandula officinalis, Ceratonia silique, Prunus dulcis, Achillea millefolium, Helianthus annuus, Rosa canina, Rosa damascene, Sideritis scardica, Sideritis syriaca, Sideritis euboea, Pinus heldreichii, Pinus nigra oder Opuntia ficus-indica.*

11. Flüssiger Extrakt, umfassend das Extraktionslösungsmittel nach einem der Ansprüche 1 bis 8 und einen kosmetisch wirksamen Inhaltsstoff, der mit dem Extraktionslösungsmittel aus Pflanzenmaterial extrahiert wurde.

12. Flüssiger Extrakt nach Anspruch 11, wobei der kosmetisch wirksame Inhaltsstoff ausgewählt ist aus einer Phenolsäure, einem Phenolsäureester, einem Flavonoid, einem Secoiridoid, einem Stilben, einem Phenolalkohol, einem Carotinoid, einem Alkaloid, einem Lipid, einem Phenylpropanoid, einem Duftstoff, einem Pigment oder einem Terpenoid.

13. Kosmetische Zusammensetzung, umfassend den flüssigen Extrakt nach Anspruch 11 oder 12 und einen kosmetisch akzeptablen Träger.

14. Kosmetische Zusammensetzung nach Anspruch 13, wobei die Zusammensetzung eine Emulsion ist.

15. Verfahren zur Herstellung des Extraktionslösungsmittels nach einem der Ansprüche 1 bis 8, wobei das Verfahren

   i) das Herstellen eines eutektischen Lösungsmittels, bestehend aus Trimethylglycin und 1,3-Propandiol in einem Molverhältnis von 1:7, durch Mischen von Trimethylglycin und 1,3-Propandiol bei 50 °C bis 70 °C, bis eine klare Lösung gebildet ist,
ii) das Zugeben von Wasser zu dem eutektischen Lösungsmittel,
iii) das Zugeben einer Cellulase zu der in b) erhaltenen Lösung, um das Extraktionslösungsmittel zu erhalten, umfasst.

**Revendications**

**1.** Solvant d'extraction destiné à l'extraction d'un ingrédient actif sur le plan cosmétique à partir de matière végétale, dans lequel le solvant comprend

a) un solvant eutectique, à une concentration comprise entre 72 % en poids et 82 % en poids du solvant d'extraction, dans lequel le solvant eutectique est constitué de triméthylglycine et de 1,3-propanediol dans un rapport molaire de 1:7,
b) de l'eau, à une concentration comprise entre 17 % en poids et 27 % en poids du solvant d'extraction, et
c) une cellulase, à une concentration comprise entre 0,005 % en poids et 0,050 % en poids du solvant d'extraction.

**2.** Solvant d'extraction selon la revendication 1, dans lequel la concentration du solvant eutectique dans le solvant d'extraction est comprise entre 75 % en poids et 79 % en poids.

**3.** Solvant d'extraction selon la revendication 1 ou la revendication 2, dans lequel la concentration du solvant eutectique dans le solvant d'extraction est comprise entre 76 % en poids et 78 % en poids.

**4.** Solvant d'extraction selon l'une quelconque des revendications précédentes, dans lequel la concentration en eau dans le solvant d'extraction est comprise entre 21 % en poids et 25 % en poids.

**5.** Solvant d'extraction selon l'une quelconque des revendications précédentes, dans lequel la concentration en eau dans le solvant d'extraction est comprise entre 22 % en poids et 24 % en poids.

**6.** Solvant d'extraction selon l'une quelconque des revendications précédentes, dans lequel la concentration en cellulase dans le solvant d'extraction est comprise entre 0,007 % en poids et 0,040 % en poids.

**7.** Solvant d'extraction selon l'une quelconque des revendications précédentes, dans lequel la concentration en cellulase dans le solvant d'extraction est comprise entre 0,008 % en poids et 0,035 % en poids.

**8.** Solvant d'extraction selon l'une quelconque des revendications précédentes, dans lequel la cellulase est un mélange d'endoglucanase, de cellobiohydrolase κα deβ-glucosidase.

**9.** Utilisation du solvant d'extraction selon l'une quelconque des revendications 1 à 8 pour l'extraction d'un ingrédient actif sur le plan cosmétique à partir d'une matière végétale.

**10.** Utilisation selon la revendication 9, dans laquelle la plante est choisie parmi *Salvia officinalis, Salvia fruticose, Helichrysum italicum, Helichrysum amorginum, Helichrysum orientale, Thymus voulgaris, Calendula officinalis, Coridothymus capitatus, Vitis vinifera, Crocus sativus. Olea europaea, Citrus medica, Lavandula officinalis, Ceratonia silique, Prunus dulcis. Achillea millefolium, Helianthus annuus, Rosa canina, Rosa damascene, Sideritis scardica, Sideritis syriaca, Sideritis euboea, Pinus heldreichii, nigra,* ou *Opuntia ficus-indica.*

**11.** Extrait liquide comprenant le solvant d'extraction selon l'une quelconque des revendications 1 à 8 et un ingrédient actif sur le plan cosmétique qui a été extrait à l'aide du solvant d'extraction à partir d'une matière végétale.

**12.** Extrait liquide selon la revendication 11, dans lequel l'ingrédient actif sur le plan cosmétique est choisi parmi un acide phénolique, un ester d'acide phénolique, un flavonoïde, un sécoiridoïde, un stilbène, un alcool phénolique, un caroténoïde, un alcaloïde, un lipide, un phénylpropanoïde, un parfum, un pigment ou un terpénoïde.

**13.** Composition cosmétique comprenant l'extrait liquide selon la revendication 11 ou la revendication 12 et un support acceptable sur le plan cosmétique.

**14.** Composition cosmétique selon la revendication 13, dans laquelle la composition est une émulsion.

**15.** Procédé de préparation du solvant d'extraction tel que défini dans l'une quelconque des revendications 1 à 8, dans lequel le procédé comprend

i) la préparation d'un solvant eutectique constitué de triméthylglycine et de 1,3-propanediol dans un rapport molaire de 1:7 en mélangeant la triméthylglycine et le 1,3-propanediol à une température comprise entre 50 °C et 70 °C jusqu'à obtention d'une solution limpide,
ii) l'ajout d'eau au solvant eutectique,
iii) l'ajout d'une cellulase à la solution obtenue en b) pour obtenir le solvant d'extraction.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 116602901

- WO 2011063080 A

**Non-patent literature cited in the description**

- **CHOI et al.** *Plant Physiol.*, August 2011, vol. 156 (4), 1701-1705
- **PAIVA et al.** *ACS Sustainable Chem. Eng*, 2014, vol. 2 (5), 1063-1071
- **SKARPALEZOS D ; DETSI A**. *Applied Sciences*, 2019, vol. 9 (19), 4169
- **PONTILLO A.R.N. et al.** *Appl. Sci.*, 2021, vol. 11, 3724, https://doi.org/10.3390/app11083724

- **RICARTE et al.** *3Biotech*, 2020, vol. 10 (9), 405
- **MAKKLIANG et al.** *Bioresour. Bioprocess.*, 2021, vol. 8, 76
- **KRISANTI et al.** *AIP Conference Proceedings*, 2019, vol. 2175, 020040